# EUROPEAN PATENT SPECIFICATION

(11) **EP 1 833 515 B1**
(45) Date of publication and mention of the grant of the patent: **28.10.2009**
(21) Application number: 05852261.6
(22) Date of filing: 23.11.2005
(51) Int. Cl.: A61L 2/03, C25B 1/26, C25B 1/30, C25B 9/00

(54) **DEVICE AND METHOD FOR INSTRUMENT STERILIZATION**
VORRICHTUNG UND VERFAHREN ZUR STERILISATION VON INSTRUMENTEN
DISPOSITIF ET PROCEDE DE STERILISATION D'INSTRUMENTS

(30) Priority: 24.11.2004 US 630877 P; 22.11.2005 US 287531
(43) Date of publication of application: 19.09.2007
(73) Proprietor: MIOX Corporation, Albuquerque, NM 87113 (US)
(72) Inventor: HERRINGTON, Rodney, Albuqueque, NM 87114 (US)
(74) Representative: Kupecz, Arpad
(86) International application number: PCT/US2005/042893
(87) International publication number: WO 2006/058282

(56) References cited:
- EP-A- 0 350 466
- WO-A-01/10215
- US-A- 4 710 233
- US-A- 6 106 691
- US-A- 6 117 285
- US-A1- 2004 195 090

## Description

### FIELD OF THE INVENTION

The present invention relates to an electrolytic method and apparatus for sterilizing instruments or other objects.

### BACKGROUND OF THE INVENTION

Note that the following discussion refers to a number of publications and references. Discussion of such publications herein is given for more complete background of the scientific principles and is not to be construed as an admission that such publications are prior art for patentability determination purposes.

Electrolytic technology utilizing dimensionally stable anodes (DSA) has been used for years for the production of chlorine and other mixed-oxidant solutions. Dimensionally stable anodes are described in U.S. Patent No. 3,234,110 to Beer, entitled "Electrode and Method of Making Same," whereby a noble metal coating is applied over a titanium substrate.

An example of an electrolytic cell with membranes is described in U.S. Patent RE 32,077 to deNora, et al., entitled "Electrode Cell with Membrane and Method for Making Same," whereby a circular dimensionally stable anode is utilized with a membrane wrapped around the anode, and a cathode concentrically located around the anode/membrane assembly.

An electrolytic cell with dimensionally stable anodes without membranes is described in U.S. Patent No. 4,761,208 to Gram, et al., entitled "Electrolytic Method and Cell for Sterilizing Water."

Various commercial electrolytic cells that have been used routinely for oxidant production may utilize a flow-through configuration that may or may not be under pressure that is adequate to create flow through the electrolytic device. Examples of cells of this configuration are described in U.S. Patent No. 6,309,523 to Prasnikar, et al., entitled "Electrode and Electrolytic Cell Containing Same," and U.S. Patent No. 5,385,711 to Baker, et al., entitled "Electrolytic Cell for Generating Sterilization Solutions Having Increased Ozone Content," and many other membrane-type cells.

In other configurations, the oxidant is produced in an open-type cell or drawn into the cell with a syringe or pump-type device, such as described in U.S. Patent No. 6,524,475 to Herrington, et al., entitled "Portable Water Disinfection System."

U.S. Patent No. 6,736,966 to Herrington, et al., entitled "Portable Water Disinfection System", describes disinfection devices that utilize, in one instance, a cell chamber whereby hydrogen gas is generated during electrolysis of an electrolyte, and provides the driving force to expel oxidant from the cell chamber through restrictive check valve type devices. In this configuration, unconverted electrolyte is also expelled from the body of the cell as hydrogen gas is generated. In an alternate configuration in the same application, hydrogen gas pressure is contained in a cell chamber during electrolysis, but the pressure within the cell chamber is limited by the action of a spring loaded piston that continues to increase the volume of the cell chamber as gas volume increases. Ultimately, a valve mechanism opens, and the spring-loaded piston fills the complete volume of the cell chamber forcing the oxidant out of the cell chamber.

In the electrolytic cells utilizing titanium substrates with noble metal coatings as the anode, the pH at the surface of the anode is typically low, on the order of approximately 3. With sufficiently high brine concentration in the electrolyte, and sufficiently low voltage potential at the anode surface, oxygen generated at the anode surface reacts to form hypochlorous acid and other chlor-oxygen compounds with no oxygen gas liberated. Typical cathodes in these electrolytic cells may be composed of titanium, noble metal coated titanium, catalyst coated titanium, nickel based allows such as Hastalloy, stainless steel, and other conductive materials impervious to high pH conditions. As the cathode, hydrogen is liberated at the cathode surface with a localized high pH value at the cathode surface. During electrolysis, the metal comprising the cathode is not oxidized or otherwise damaged during electrolysis despite the production of hydrogen at the cathode surface. Over time, titanium hydride can form at the surface of a bare titanium cathode which may cause stress concentrations in the cathode surface. To preclude this hydride formation, noble metal or catalyst coatings can be applied to the cathode surface to prevent titanium hydride from forming on the cathode surface when the cathode substrate comprises titanium.

In a paper by Christine Rabinovitch, entitled "Electrochemical Control of Staphylococcus epidermidis Biofilms", published in the Proceedings of the Winter 2004 CBE Technical Advisory Conference, February 5-6, 2004 (Montana State University, Center for Biofilm Engineering), an electrolytic method is described whereby a metallic coupon with a biofilm media grown on the surface of the metallic coupon is utilized as the anode and/or cathode in an electrolytic reaction. When the metallic coupon is utilized as the cathode, hydrogen liberated at the surface of the cathode forces partial or complete discharge and removal of the biofilm from the metallic cathode surface. Sodium hydroxide with a high pH value that is produced at the cathode surface may also play a role destruction of the biofilms. The author presents the limitations of corrosion and oxidation of the anode and/or cathode coupon during the electrolytic process and questions the efficacy of chlorine species liberated at the anode surface as the source of destruction of biofilm at the anode.

An anti-biofouling system is described in U.S. Patent No. 6,514,401 by Chyou, et al whereby a graphite powder with binder is formed on an underwater structure immersed in seawater. The conductive surface acts as an anode or cathode when electrical power is applied to form an electrolytic surface whereby organisms are prohibited from forming on the structure.

A medical instrument sterilizing system is described in U.S. Patent No. 6,056,866 by Maeda, et al whereby an electrolytic solution is generated in an electrolytic cell, and a pumping device circulates the disinfectant to a separate instrument tray where the instruments are sterilized.

### SUMMARY OF THE INVENTION

The present invention is an instrument sterilization apparatus comprising at least two electrodes wherein at least one electrode comprises at least one cathode and at least one electrode comprises at least one anode, the at least one cathode comprising a tray for holding and providing electrical contact to an instrument and the at least one anode comprising a tank; at least one insulator for separating the anode and the cathode; and electrolyte disposed in the tank; wherein an electrical potential between the anode and the cathode causes an electrical charge to pass through the electrolyte, thereby generating at least one oxidant in the electrolyte. The instrument preferably contacts the tray, which is preferably porous.

The apparatus preferably further comprises an electrolyte storage container which is preferably replaceable and which preferably further comprises a quick disconnect valve for allowing flow of the electrolyte to the tank only when the container is attached to the apparatus. The apparatus preferably further comprises a microprocessor circuit that identifies the electrolyte storage container with the apparatus or measures a remaining volume of the electrolyte in the storage container. The apparatus optionally further comprises a brine generating device. The apparatus preferably further comprises an oxidant residual measuring device. The apparatus also preferably further comprises a vent or reactor for eliminating hydrogen gas generated during sterilization. The apparatus preferably further comprises a tube for circulating electrolyte to the interior of an instrument.

The present invention is also a method for sterilizing and instrument, the method comprising the steps of disposing electrolyte in a tank; placing an instrument in a tray, the tray electrically insulated from the tank; providing an electrical potential between the tank and the tray so that the tank becomes an anode in an electrolytic reaction and the tray becomes a cathode in the reaction; and generating at least one oxidant in the electrolyte. The method preferably comprises placing the instrument in electrical contact with the tray, in which case hydrogen gas is preferably generated at the surface of the instrument.

The method preferably further comprises the step of measuring a value of a characteristic of the electrolyte, and optionally adjusting the electrical potential so that the value remains within a desired range. The method preferably further comprises the step of circulating electrolyte to an interior surface or interior component of the instrument. The method preferably further comprises the step of eliminating hydrogen gas generated during the reaction by venting or reacting the hydrogen gas. The method preferably further comprises the step of rinsing the instrument with a sterile solution. The method preferably further comprises the step of drying the instrument with heated air.

An object of the present invention is to simplify sterilizing apparatuses by using sterilizing trays as the anode and cathode together in one unit.

A further object of the present invention is to utilize on or more of hydrogen formation, chlorine or mixed oxidant formation, and sodium hydroxide formation to sterilize instruments.

An advantage of the present invention is that the medical instruments or other devices requiring sterilization are preferably utilized as the anode and/or cathode in the electrolytic process, thus enhancing the sterilizing properties of the system.

Other objects, advantages and novel features, and further scope of applicability of the present invention will be set forth in part in the detailed description to follow, taken in conjunction with the accompanying drawings, and in part will become apparent to those skilled in the art upon examination of the following, or may be learned by practice of the invention. The objects and advantages of the invention may be realized and attained by means of the instrumentalities and combinations particularly pointed out in the appended claims.

### BRIEF DESCRIPTION OF THE SEVERAL VIEWS OF THE DRAWINGS

The accompanying drawings, which are incorporated into and form a part of the specification, illustrate several embodiments of the present invention and, together with the description, serve to explain the principles of the invention. The drawings are only for the purpose of illustrating a preferred embodiment of the invention and are not to be construed as limiting the invention. In the drawings:
FIG. 1 is a system view of an embodiment of the present invention where the device to be sterilized is in electrical contact with a cathode tray.
FIG. 2 is a view of the quick disconnect electrolyte cartridge of the present invention.
FIG. 3 is a view of the present invention comprising optional heater sterile water rinse modules.

### DETAILED DESCRIPTION OF THE INVENTION

The present invention comprises an electrolytic device and method for generation of hydrogen gas at the cathode surface and oxidants produced at both the anode and cathode, which are utilized to expel contaminants such as biofilms and to disinfect surfaces, such as for sterilizing instruments and other devices. The present invention preferably utilizes the features of stable anodes and cathodes, formation of hydrogen formed at the cathode surface, formation of sodium hydroxide generated at the cathode surface, and formation of chlorine and mixed-oxidant species generated in a low pH environment at the anode surface as the basis for sterilizing instruments or objects. As used throughout the specification and claims, to "sterilize" means to sterilize, disinfect, or otherwise clean. As used throughout the specification and claims, "instrument" means any object or device to be sterilized, including but not limited to surgical instruments, endoscopes, utensils, and the like.

In the present invention the instrument to be sterilized is preferably used as the cathode in an electrolytic process. Hydrogen liberated at the cathode (instrument) surface discharges all materials, including but not limited to biofilms, from the instrument surface. Sodium hydroxide at high pH acts as a caustic substance to disinfect the device. Chlorine liberated at the anode surface at low pH (acidic) disinfects microorganisms on the device surface, or creates a disinfectant in the solution to kill microorganisms on the device surface and microorganisms or organic material in the electrolyte fluid. The instrument is preferably at least partially electrically conductive, and more preferably at least partially metallic. Instruments preferably comprise titanium, Hastalloy, stainless steel, conductive plastic, or other caustic (high pH) resisting materials. Plastic components of the instruments are optionally impregnated with titanium, hastalloy, stainless, carbon, or other conductive filings that then make the plastic electrically conductive to facilitate hydrogen formation at the surface of the plastic. An oxidant solution pump is preferably utilized to pump oxidant to the internal components and surfaces of instruments such as endoscopes. A hydrogen vent and recombiner device preferably converts hydrogen liberated in the electrolysis process, and oxygen from the atmosphere, to water vapor to mitigate the dangers associated with hydrogen gas.

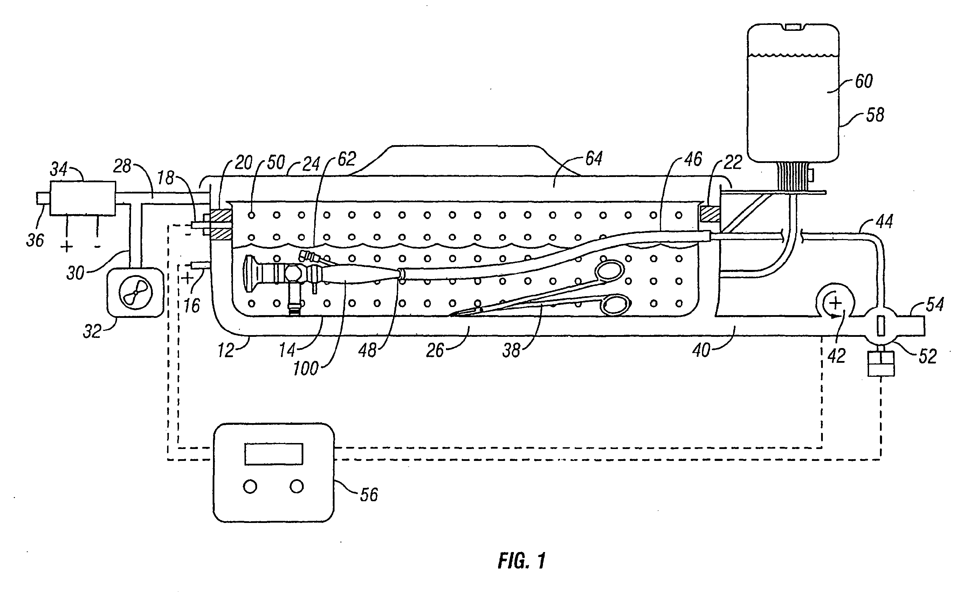

Referring to FIG. 1, tank **12** preferably acts as the anode and preferably comprises a catalytic coating on its interior surface, which contains electrolyte **62.** Inner tray **14** preferably comprises the primary cathode. Inner tray **14** preferably comprises a suitable cathode material, preferably titanium, and further preferably comprises perforations **50** that allow electrolyte 62 to freely flow around both the outside and the inside of inner tray **14.** Inner tray **14** is optionally removeable. By application of the appropriate positive (preferably direct current) voltage and current density to tank **12** and negative (preferably direct current) voltage and current density to inner tray **14,** the electrolyte is preferably converted to chlorine-based mixed-oxidant species within electrolysis space **26.**

When instrument **38** is placed within inner tray **14,** instrument **38** preferably comes into electrical contact with inner tray **14** and thereby also acts as a cathode in the electrolysis process. Alternatively, inner tray **14** is not used, and negative DC electrical power is directly applied to instrument **38** via cathode electrical conductor **18;** in this case, instrument **38** preferably rests in a perforated plastic mesh, perforated Teflon liner, or the like, or rests on insulating supports, within tank **12** so there is no electrical contact between tank **12** (the anode) and instrument **38** (the cathode).

Instrument **38** is preferably constructed of a conducting material, or can be produced from a plastic material with additives to make the plastic components electrically conductive. In the electrolysis process of the present invention, hydrogen is liberated at the surface of the cathode. Hydrogen bubbles preferably act as a physical scrubbing agent to remove material from the surface of instrument **38.** Such material includes but is not limited to organic materials, biological materials, biofilms, or other organic matter. The organic contaminants are then transferred to bulk electrolyte **62,** which is preferably concurrently converted to a chlorine-based mixed oxidant solution. The mixed-oxidant solution then acts as a disinfecting solution to destroy the organic contaminants within bulk electrolyte **62.**

Tank **12**, which preferably serves as the anode in the system, preferably mechanically supports and is electrically insulated from inner tray **14,** which preferably serves as the cathode, by a plurality of insulators **20, 22.** Alternatively one insulator may be disposed on the inside of tank **12,** for example in a ring shape, for supporting inner tray **14.** Although inner tray **14** is preferably attached to insulators **20, 22,** it may optionally removeably rest on insulators **20, 22,** optionally via a lip on the tray or by some other means. Positive DC electrical power is preferably applied to tank **12** via anode electrical conductor **16.** Negative DC electrical power is preferably applied to inner tray **14** via cathode electrical conductor **18.** Power to tank **12** and inner tray **14** is preferably regulated and controlled by controller **56,** which also preferably controls the system.

The present system and method preferably comprise a batch process that maintains a desired residual oxidant value, preferably a residual chlorine value, within electrolyte **62.** The sterilizing device of the present invention preferably comprises an oxidant residual monitoring device **70,** which preferably comprises an oxidation reduction potential (ORP) sensor or a chlorine sensor, preferably mounted on an integrated circuit device (for example, a chlorine sensor-on-a-chip). The ORP value may optionally be adjusted for variations in temperature and pH of electrolyte **62.**

Monitoring device **70** may be then used in a feedback system for controlling the electrolytic operation of the system. Oxidant residual monitoring device **70** monitors the chlorine residual value, preferably via controller **56.** If the chlorine residual value is below the desired value, controller **56** provides additional power to tank **12** and inner tray **14** thereby producing additional oxidant in electrolyte **62.** In this mode of operation, neither the oxidant demand nor the volume or fluid level of electrolyte **62** are important to maintaining the desired chlorine or other oxidant residual value. If the chlorine residual value is not sufficient, controller **56** continues making oxidant until the desired chlorine residual is maintained.

Circulation of electrolyte **62** in the device may be desirable. For example, internal components of endoscope **100** used for some medical procedures may become contaminated. By circulating electrolyte **62** to the internal surface of endoscope **100,** the internal components of endoscope **100** are cleaned in the same manner as the exterior surface of endoscope **100.** To facilitate circulation of electrolyte **62,** pump **42** preferably transfers electrolyte **62** through passage **40** from tank **12** and through 3-way valve **52** and passage **44** into tube **46** and out of tube end **48.** Tube end **48** is preferably connected to a hollow passage of a medical instrument, such as endoscope **100,** so that sterilizing fluid can be flushed through the internal components of the medical instruments. When the flushing cycle is complete, electrolyte **62** may optionally be discarded from the sterilizing tray by switching the valve position of 3-way valve **52** and thereby discharging electrolyte through outlet **54.** Pump **42** and 3-way valve **52** are preferably controlled by controller **56.**

Electrolysis typically generates hydrogen gas at the cathode electrode. Hydrogen gas is explosive at a wide range of pressures. Only below a concentration of approximately 4.1 % hydrogen in the atmosphere, or above a concentration of about 74.2% hydrogen in the atmosphere, is the gas mixture not combustible. Thus, for proper safety, hydrogen containment or elimination is desirable. In the present invention, hydrogen gas accumulates in the upper space **64** of the sterilizing unit and is contained there by device cover **24.** The hydrogen gas is preferably transferred from upper space **64** via passage **28** to catalytic recombiner **34,** which is preferably utilized to burn hydrogen with oxygen to produce water vapor, which is preferably discharged out of port **36.** Normal atmospheric air is preferably drawn into blower **32** and transferred through passage **30** to provide the oxygen source for reacting with hydrogen within catalytic combiner **34.** In this manner, hydrogen is destroyed and is no longer available as a fuel source for an explosive event. Alternatively, a vent pipe transfers hydrogen from upper space **64** to a ventilation duct and discharges it outside of the room or facility that houses the sterilizing device.

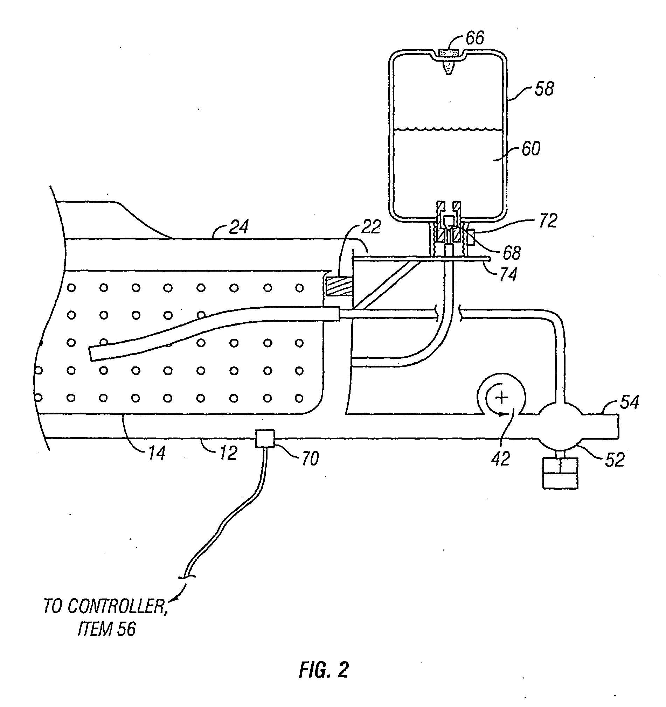

Electrolyte **62** preferably comprises a sodium chloride brine solution. Other halide salts may alternatively be utilized to produce electrolyte **62.** For medical applications, a preferred source of brine solution is .9% saline Ringers solution. Pre-made electrolyte solution 60 is preferably stored within electrolyte storage container **58** for use with the present invention. Referring to FIG. 2, electrolyte storage container **58** is optionally attached to the sterilizing tray via support **74** and is preferably quickly removable from the system by means of quick disconnect self-sealing valve **68** for subsequent replacement by a new electrolyte storage container. Alternatively, electrolyte storage container **58** may be refillable. Electrolyte storage container **58** preferably comprises vent valve **66** that allows the introduction of air into electrolyte storage container **58** as pre-made electrolyte solution **60** is drawn out of container **58** thereby avoiding negative pressure in container **58.**

Container **58** optionally comprises microchip device **72** that identifies container **58** with the total system, and preferably provides for electronic monitoring of the volume of the contents of container **58** based on the number of cycles of the system or another property. Electrolyte storage container **58** is optionally replaced with a brine generating device. Such brine generating device is preferably filled with salt, preferably a halogen salt, and mixes water with the halogen salt to produce a liquid brine solution. The liquid brine solution performs as electrolyte **62.**

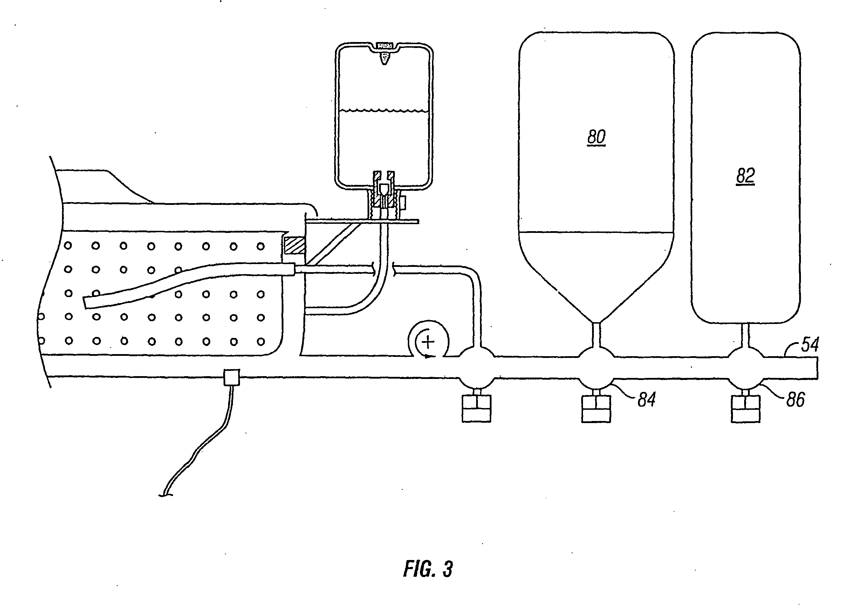

As shown in FIG. 3, the sterilizing system optionally comprises heater/dryer module **80** and sterile water purge module **82.** In this embodiment, the initial sterilizing cycle is preferably followed by draining of electrolyte via 3-way valve **52** through outlet **54** and subsequent closure of 3-way valve **52.** In the second step, 3-way purge valve **86** is opened to allow transfer of sterile water, or another sterile solution, from sterile water purge module **82** for rinsing tank **12;** 3-way purge valve **86** preferably prevents the sterile water from exiting via outlet **54.** After purging, 3-way purge valve **86** is preferably opened to drain sterile water via outlet **54.** In the final step, 3-way valve **84** is preferably opened to allow heated dry air from heater/dryer module **80** to dry the instruments within tank **12.**

The present invention optionally comprises one or more oxidant storage containers and at least one port for injecting one or more oxidants into an instrument, including but not limited to a closed fluid body, an open fluid body, a pipe with fluid flowing therein, a sump, a basin, a trough, or a plenum.

The present invention is particularly applicable to medical instrument sterilization. However, it will be obvious to those versed in the art that this invention can be utilized in a variety of applications where other objects or devices need to be sterilized or otherwise cleaned, including but not limited to dishwashing machines, cabinets, or other configurations.

## Claims

1. An instrument sterilization apparatus **characterised by**:
at least two electrodes wherein at least one electrode comprises at least one cathode and at least one electrode comprises at least one anode, said at least one cathode comprising a tray (14) for holding and providing electrical contact to an instrument and said at least one anode comprising a tank (12);
at least one insulator (20, 22) for separating said anode and said cathode; and
electrolyte (62) disposed in said tank (12);
wherein an electrical potential between said anode and said cathode causes an electrical charge to pass through said electrolyte (62), thereby generating at least one oxidant in the electrolyte (62).

2. The apparatus of claim 1 further **characterised by** an electrolyte storage container (58).

3. The apparatus of claim 2 **characterised in that** said electrolyte storage container (58) is replaceable.

4. The apparatus of claim 3 **characterised in that** said electrolyte storage container (58) further comprises a quick disconnect valve (68) for allowing flow of said electrolyte to said tank (12) only when said container is attached to said apparatus.

5. The apparatus of claim 2 further **characterised by** a microprocessor circuit that identifies said electrolyte storage container (58) with said apparatus or measures a remaining volume of said electrolyte in said storage container (58).

6. The apparatus of claim 1 further **characterised by** a brine generating device.

7. The apparatus of claim 1 **characterised in that** the instrument contacts said tray (14).

8. The apparatus of claim 1 further **characterised by** an oxidant residual measuring device (70).

9. The apparatus of claim 1 further **characterised by** a vent or reactor for eliminating hydrogen gas generated during sterilization.

10. The apparatus of claim 1 **characterised in that** said tray (14) is porous.

11. The apparatus of claim 1 further **characterised by** a tube for circulating electrolyte (62) to the interior of an Instrument.

12. A method for sterilizing and instrument, the method **characterised by** the steps of:
disposing electrolyte (62) in a tank (12);
placing an instrument in a tray (14), the tray electrically insulated from the tank (12);
providing an electrical potential between the tank (12) and the tray (14) so that the tank (12) becomes an anode in an electrolytic reaction and the tray becomes a cathode in the reaction; and
generating at least one oxidant in the electrolyte (62).

13. The method of claim 12 **characterised in that** the placing step comprises placing the instrument in electrical contact with the tray (14).

14. The method of claim 13 further **characterised by** the step of generating hydrogen gas at the surface of the instrument.

15. The method of claim 12 further **characterised by** the step of measuring a value of a characteristic of the electrolyte (62).

16. The method of claim 15 further **characterised by** the step of adjusting the electrical potential so that the value remains within a desired range.

17. The method of claim 12 further **characterised by** the step of circulating electrolyte (62) to an interior surface or interior component of the Instrument.

18. The method of claim 12 further **characterised by** the step of eliminating hydrogen gas generated during the reaction by venting or reacting the hydrogen gas.

19. The method of claim 12 further **characterised by** the step of rinsing the instrument with a sterile solution.

20. The method of claim 12 further **characterised by** the step of drying the instrument with heated air.

## Patentansprüche

1. Vorrichtung zur Sterilisation von Instrumenten, **gekennzeichnet durch**:
mindestens zwei Elektroden, wobei wenigstens eine Elektrode wenigstens eine Kathode und wenigstens eine Elektrode wenigstens eine Anode umfasst, und wobei die genannte wenigstens eine Kathode einen Boden (14) zum Halten eines Instruments und zur Herstellung eines elektrischen Kontakts zu einem Instrument und die genannte wenigstens eine Anode einer Wanne (12) umfaßt;
wenigstens einen Isolator (20, 22) zur Trennung der Anode und Kathode; und Elektrolyt (62) in der Wanne (12);
wobei ein elektrisches Potential zwischen Anode und Kathode eine elektrische Ladung veranlasst, **durch** den Elektrolyten (62) zu wandern, wodurch mindestens ein Oxidationsmittel in dem Elektrolyten (62) erzeugt wird.

2. Vorrichtung nach Anspruch 1, **gekennzeichnet durch** einen Elektrolytvorratsbehälter (58).

3. Vorrichtung nach Anspruch 2,**dadurch gekennzeichnet, dass** der Elektrolytvorratsbehälter (58) auswechselbar ist.

4. Vorrichtung nach Anspruch 3, **dadurch gekennzeichnet, dass** der Elektrolytvorratsbehälter (58) ein Schnelltrennventil (68) aufweist, welches einen Elektrolytfluss zur Wanne (12) nur zulässt, wenn der Behälter mit der Vorrichtung verbunden ist.

5. Vorrichtung nach Anspruch 2, **gekennzeichnet durch** einen Mikroprozessorschaltkreis, der den Elektrolytvorratsbehälter (58) mit der Vorrichtung identifiziert oder ein Restvolumen des Elektrolyts im Vorratsbehälter (58) misst.

6. Vorrichtung nach Anspruch 1, **gekennzeichnet durch** eine Einrichtung zur Laugenerzeugung.

7. Vorrichtung nach Anspruch 1, **dadurch gekennzeichnet, dass** die Instrumente den Boden (14) berühren.

8. Vorrichtung nach Anspruch 1, **gekennzeichnet durch** eine Messeinrichtung für den Oxidationsmittelrest.

9. Vorrichtung nach Anspruch 1, **gekennzeichnet durch** einen Auslass oder Reaktor zur Entfernung von während der Sterilisation gebildetem Wasserstoff.

10. Vorrichtung nach Anspruch 1, **dadurch gekennzeichnet, dass** der Boden (14) porös ist.

11. Vorrichtung nach Anspruch 1, **gekennzeichnet durch** ein Rohr zur Elektrolytzirkulation ins Innere eines Instruments.

12. Verfahren zur Sterilisation von Instrumenten, **gekennzeichnet durch** die Schritte:
Bereitstellung eines Elektrolyts (62) in einer Wanne (12),
Platzierung eines Instruments auf einem Boden (14), der von der Wanne elektrisch isoliert ist,
Bereitstellung eines elektrischen Potentials zwischen Wanne und Boden, so, dass die Wanne in einer elektrolytischen Reaktion zur Anode und der Boden zur Kathode wird, und Erzeugung mindestens eines Oxidationsmittels in dem Elektrolyten (62).

13. Verfahren nach Anspruch 12, **dadurch gekennzeichnet, dass** der Platzierungsschritt die Platzierung des Instruments in elektrischem Kontakt mit dem Boden (14) umfasst.

14. Verfahren nach Anspruch (13), **gekennzeichnet durch** den Schritt der Erzeugung von Wasserstoffgas an der Oberfläche des Instruments.

15. Verfahren nach Anspruch 12, **gekennzeichnet durch** den Schritt einer Messung eines Wertes einer Eigenschaft des Elektrolyten (62).

16. Verfahren nach Anspruch 15, **gekennzeichnet durch** den Schritt einer Einstellung des elektrischen Potentials in der Weise, dass der Wert innerhalb eines gewünschten Bereichs bleibt.

17. Verfahren nach Anspruch 12, **gekennzeichnet durch** den Schritt der Zirkulation des Elektrolyts (62) zu einer inneren Oberfläche oder einer inneren Komponente des Instruments.

18. Verfahren nach Anspruch 12, **gekennzeichnet durch** den Schritt der Entfernung von Wasserstoffgas, das während der Reaktion gebildet wird, **durch** Abziehen oder Reaktion des Wasserstoffgases.

19. Verfahren nach Anspruch 12, **gekennzeichnet durch** den Schritt des Spülens des Instruments mit einer sterilen Lösung.

20. Verfahren nach Anspruch 12, **gekennzeichnet durch** den Schritt des Trocknens des Instruments mit erhitzter Luft.

## Revendications

1. Appareil de stérilisation d'instruments, **caractérisé par** :
au moins deux électrodes, dans lequel au moins une électrode comprend au moins une cathode et au moins une électrode comprend au moins une anode, ladite au moins une cathode comprenant un bac (14) destiné à recevoir un instrument et établir le contact électrique avec lui et ladite au moins une anode comprend une cuve (12) ;
au moins un isolateur (20, 22), destiné à séparer ladite anode et ladite cathode ; et
un électrolyte (62) placé dans ladite cuve (12) ;
dans lequel un potentiel électrique entre ladite anode et ladite cathode commande le passage d'une charge électrique au travers dudit électrolyte (62), ce qui produit au moins un oxydant dans l'électrolyte (62).

2. Appareil selon la revendication 1, **caractérisé en outre par** un conteneur de stockage d'électrolyte (58).

3. Appareil selon la revendication 2, **caractérisé en ce que** le conteneur de stockage d'électrolyte (58) peut être remplacé.

4. Appareil selon la revendication 3, **caractérisé en ce que** ledit conteneur de stockage d'électrolyte (58) comprend en outre une vanne de déconnexion rapide (68) destinée à ne permettre un écoulement dudit électrolyte vers ladite cuve (12) que lorsque ledit conteneur est fixé sur ledit appareil.

5. Appareil selon la revendication 2, **caractérisé en outre par** un circuit microprocesseur qui identifie ledit conteneur de stockage d'électrolyte (58) auprès dudit appareil ou qui mesure un volume résiduel dudit électrolyte dans ledit conteneur de stockage (58).

6. Appareil selon la revendication 1, **caractérisé par** un dispositif de production de saumure.

7. Appareil selon la revendication 1, **caractérisé en ce que** l'instrument entre en contact avec ledit bac (14).

8. Appareil selon la revendication 1, **caractérisé par** un dispositif de mesure d'oxydant résiduel (70).

9. Appareil selon la revendication 1, **caractérisé par** un orifice d'évacuation ou un réacteur afin d'éliminer l'hydrogène gazeux produit pendant la stérilisation.

10. Appareil selon la revendication 1, **caractérisé en ce que** ledit bac (14) est poreux.

11. Appareil selon la revendication 1, **caractérisé en outre par** un tube destiné à faire circuler l'électrolyte (62) à l'intérieur d'un instrument.

12. Procédé de stérilisation d'un instrument, le procédé étant **caractérisé par** les étapes consistant à :
mettre de l'électrolyte (62) dans une cuve (12);
placer un instrument dans un bac (14), le bac étant électriquement isolé de la cuve (12) ;
fournir un potentiel électrique entre la cuve (12) et le bac (14) afin que la cuve (12) devienne une anode dans une réaction électrolytique et le bac devienne une cathode dans la réaction ; et
produire au moins un oxydant dans l'électrolyte (62).

13. Procédé selon la revendication 12, **caractérisé en ce que** l'étape de placement comprend l'étape consistant à placer l'instrument en contact électrique avec le bac (14).

14. Procédé selon la revendication 13, **caractérisé en outre par** l'étape consistant à produire de l'hydrogène gazeux à la surface de l'instrument.

15. Procédé selon la revendication 12, **caractérisé en outre par** l'étape consistant à mesurer une valeur d'une caractéristique de l'électrolyte (62).

16. Procédé selon la revendication 15, **caractérisé en outre par** l'étape consistant à ajuster le potentiel électrique de manière à ce que la valeur reste dans une plage souhaitée.

17. Procédé selon la revendication 12, **caractérisé en outre par** l'étape consistant à faire circuler l'électrolyte (62) vers une surface intérieure ou un composant intérieur de l'instrument .

18. Procédé selon la revendication 12, **caractérisé en outre par** l'étape consistant à éliminer l'hydrogène gazeux produit pendant la réaction en évacuant ou en faisant réagir l'hydrogène gazeux.

19. Procédé selon la revendication 12, **caractérisé en outre par** l'étape consistant à rincer l'instrument avec une solution stérile.

20. Procédé selon la revendication 12, **caractérisé en outre par** l'étape consistant à sécher l'instrument à l'aide d'air chaud.
